(19) Europäisches Patentamt
European Patent Office
Office européen des brevets

(11) **EP 3 115 782 B1**

(12) **EUROPEAN PATENT SPECIFICATION**

(45) Date of publication and mention of the grant of the patent:
**26.12.2018 Bulletin 2018/52**

(51) Int Cl.:
***G01N 33/497*** *(2006.01)* ***G01N 33/68*** *(2006.01)*

(21) Application number: **16178332.9**

(22) Date of filing: **07.07.2016**

(54) **METHOD FOR DETERMINING PROTEIN OXIDATION**

VERFAHREN ZUR BESTIMMUNG DER PROTEINOXIDATION

PROCÉDÉ PERMETTANT DE DÉTERMINER L'OXYDATION DE PROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2015 EP 15176054**

(43) Date of publication of application:
**11.01.2017 Bulletin 2017/02**

(73) Proprietor: **Hanze Nutrition B.V.**
**9606 PE Kropswolde (NL)**

(72) Inventor: **Vonk, Roel J.**
**9606 PE Kropswolde (NL)**

(74) Representative: **Gille Hrabal**
**Brucknerstrasse 20**
**40593 Düsseldorf (DE)**

(56) References cited:
**US-A1- 2008 281 194**

- **FEDERICA CAMIN ET AL: "Influence of dietary composition on the carbon, nitrogen, oxygen and hydrogen stable isotope ratios of milk", RAPID COMMUNICATIONS IN MASS SPECTROMETRY., vol. 22, no. 11, 15 June 2008 (2008-06-15) , pages 1690-1696, XP055219150, GB ISSN: 0951-4198, DOI: 10.1002/rcm.3506**
- **Klaus Wetzel ET AL: "13C-Breath Tests in Medical Research and Clinical Diagnosis", , 1 May 2005 (2005-05-01), XP055096455, Retrieved from the Internet: URL:http://www.fan-gmbh.de/docs/13c-breath tests.pdf [retrieved on 2014-01-15]**
- **EVENEPOEL P ET AL: "DIGSTIBILITY OF COOKED AND RAW EGG PROTEIN IN HUMANS AS ASSESSED BY STABLE ISOTOPE TECHNIQUES", THE JOURNAL OF NUTRITION, AMERICAN SOCIETY FOR NUTRITION, US, vol. 128, no. 10, 1 January 1998 (1998-01-01), pages 1716-1722, XP008061476, ISSN: 0022-3166**
- **GHOOS Y ET AL: "Use of naturally <13>C-enriched substrates for the study of carbohydrate and protein assimilation by means of <13>CO2-breath tests", APPLIED RADIATION AND ISOTOPES, INTERNATIONAL JOURNAL OFRADIATION APPLICATIONS AND INSTRUMENTATION, PART A, PERGAMON PRESS LTD, GB, vol. 39, no. 6, 1 January 1988 (1988-01-01), page 584, XP024719963, ISSN: 0883-2889, DOI: 10.1016/0883-2889(88)90335-8 [retrieved on 1988-01-01]**
- **Y Ghoos ET AL: "C protein breath tests", Gut, 1 November 1998 (1998-11-01), page S23, XP055219310, London Retrieved from the Internet: URL:http://gut.bmj.com/content/43/suppl_3/ S23.full.pdf**

EP 3 115 782 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The invention relates to a method of quantitatively determining the protein oxidation in an individual from breath test samples. The invention further relates to new isolated natural $^{13}C$ enriched milk substrates, which may be used for deriving the breath test samples for the method of the present invention.

INTRODUCTION:

Easy and reliable assessment of the protein status of an individual would be highly valuable in clinical and epidemiological research. Direct measurement of protein oxidation in breath has been found to be a parameter directly related to this.

It has already been described that in principle protein oxidation reactions can be determined in breath test samples using so called $^{13}C$ breath tests. The general principle underlying the $^{13}C$ breath tests is based on the observation of $^{13}CO_2$ enrichment in exhaled breath after administration (oral or intravenous) and metabolism of a substrate labelled with the stable $^{13}C$ carbon isotope. The $^{13}C$ carbon is a stable isotope with a natural abundance of 1.109 % (compared to $^{12}C$ natural abundance of 98.89 %). The $^{13}C$ carbon isotope is free of radiation (in contrast to the $^{14}C$ carbon isotope) and is thus safe for use in living individuals such as animals, humans and in particular children, pregnant women and ill individuals (patients). The $^{13}C$ isotope analysis in breath can be carried out using Isotope Ratio Mass Spectrometry (IRMS).

For this purpose at certain intervals before and after ingestion of a $^{13}C$ enriched milk substrate, breath samples are collected and analyzed with respect to the $^{13}C$ content. The rate-limiting step of the ingestion of the marker ($^{13}C$ substrate) until its appearance in the metabolic endproduct $^{13}CO_2$ in the breath may depend on hydrolyses (digestion) of the substrate in the intestinal lumen, its transport processes or other metabolic steps. Finally, from the initially administered substrate $^{13}CO_2$ is released, which is exhaled via the breath.

The so far applied breath tests are based on the hypothesis that besides the focused, rate-limiting step all other metabolic processes proceed negligibly quick or at least at a constant rate. Depending on the choice of the appropriate $^{13}C$ labelled substrate transport and digestive processes, absorption and oxidation processes or (bacterial) enzymatic activities can be analyzed accordingly by means of $^{13}C$ breath tests. Meanwhile various $^{13}C$ labelled test substrates for various diagnostic problems are available.

Braden et. al; Deutsches Arzteblatt 2003; 100 (51-52) provide an overview of various $^{13}C$ breath test applications and mention inter alia a test for examining the trypsin activity in a $^{13}C$ protein derived breath test using $^{13}C$ labelled egg white.

Wetzel and Fischer; Fischer Analysen Instrumente GmbH, Leipzig; 2005 mention in their publication "$^{13}C$ Breath Tests in Medical Research and Clinical Diagnosis" $^{13}C$ breath tests with protein-rich natural substrates, their general suitability for examining resorption and metabolic degradation of proteins and particularly its application for measuring the pancreatic trypsin activity in the small intestine.

Boirie, Y. et al., Physiology 1997, vol. 94, pp. 14930-14935 and various other authors such as for example Evenepoel et al., J Nutr 1997, 127: 327-331 and Evenepoel et al., J Nutr 1998, 128: 1716-1722 described $^{13}C$ protein derived breath tests using a high-enriched egg protein substrate with $^{13}C$ labelled leucine, therewith observing the leucine kinetics.

Similarly, Ghoos Y. et al. "Use of Naturally 13C-enriched Substrates for the Study of Carbohydrate and Protein Assimilation by Means of 13CO2 Breath Tests", Applied Radiation and Isotopes, Int. J. of Radiation Applications and Instrumentation, Part A, Vol. 39, No. 6, Page 584, 1988 and Y. Ghoos and B. Beaufrere "13C protein breath tests", Gut 1998; 43 (Suppl. 3), S23-S24 described $^{13}C$ protein derived breath tests using high-enriched egg protein and milk protein such as casein with $^{13}C$ labelled leucine, therewith observing the leucine kinetics.

Metges et al., British Journal of Nutrition 1992, 67, 43-55 described the determination of $^{13}C$ recovery from $^{13}C$ labelled milk diets via $^{13}C$ breath tests in sucking calves.

Federica Camin et al. "Influence of dietary composition on the carbon, nitrogen, oxygen and hydrogen stable isotope ratios of milk", Rapid Commun. Mass Spectrom. 2008; 22: 1690-1696 describe multi-element isotope ratio investigations of milk and casein derived from cows fed with a mixed $C_3$ and $C_4$ diet on the basis of maize.

US 2008/0281194 describes methods for assessing digestive functions using a breath test using label incorporated proteins, carbohydrates and lipids, wherein the used labelled proteins are freeze-dried labelled egg protein substrates.

However, none of the so far described evaluations provides a method for quantitatively determining the protein oxidation by its direct measurement in breath test samples, derived from low-enriched $^{13}C$ labelled protein substrates, with sufficient precision to allow substantiated individual overall protein oxidation results, which may be used as a basis for a substantiated correlation to an individual protein status. For example, measuring $^{13}C$ labelled leucine only does not take into account that the oxidation of this essential amino acid does not represent the oxidation characteristics of

all amino acids in the initial protein substrate.

Further, the method of high-enrichment of substrates as used for preparing said $^{13}C$ labelled leucine substrates is expensive.

Measurement of the $^{13}C$ recovery from $^{13}C$ labelled milk diets in calves, which belong to the group of ruminants with a very specialized stomach and digestion system, is not suitable for adequately providing results for quantitatively determining the protein oxidation with sufficiently high precision either and thus does not provide any basis for a reasoned correlation to the complex protein status of the individual, such as in particular in humans.

OBJECT:

The objective of the present invention was to provide a method for determining protein oxidation from $^{13}C$ breath test samples, which avoids the aforementioned disadvantages. A particular objective of the present invention was to provide a simple, cheap and highly reproducible method of quantitatively determining the protein oxidation in an individual with an improved method for determining the individual protein oxidation rates with high and improved precision. The new and improved method should further particularly be sufficiently precise to allow a correlation from the determined protein oxidation to the individual protein status. Finally, the new method should be particularly suitable to determine the protein oxidation in breath test samples of mammals, particularly in humans.

DETAILED DESCRIPTION OF THE INVENTION:

The present invention is defined in the appended claims. The inventors of the present invention surprisingly found that the aforementioned disadvantages can be solved by applying the new method for quantitatively determining the protein oxidation in an individual as described herein. The new method turned out to be particularly suitable, when carried out with the new $^{13}C$ enriched milk substrate according to the present invention. In one aspect, the new method is in particular based on a $^{13}C$ protein derived breath test evaluation using overall low-enriched labelling of all amino acids in the underlying $^{13}C$ enriched milk substrate. This allows the determination of protein oxidation in the breath test samples on the basis of all metabolized proteins and therefore more precisely reflects the ultimate metabolic fate of all amino acids instead of only $^{13}C$ leucine labelled milk proteins, thus allowing a reasoned correlation to the individual protein status.

The protein oxidation process, which is the underlying metabolic process of the method of the present invention, can be illustrated as follows:

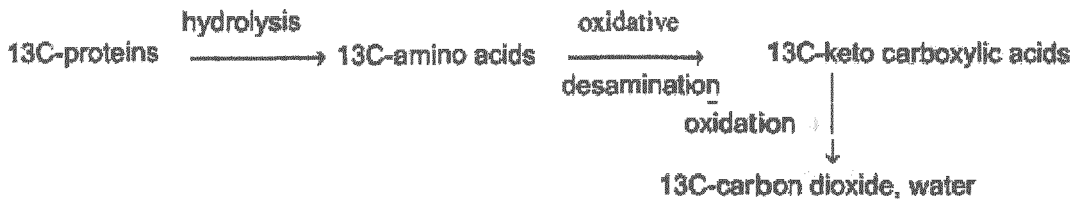


or simply by:

$$^{13}C\ \text{substrate} + O_2 \rightarrow H_2O + {}^{13}CO_2$$

Now, breath of an individual contains all kinds of metabolites of which $CO_2$ is a major component. $CO_2$ is formed when a substrate is oxidized. Breath, including $CO_2$, can be captured and analyzed. By using a $^{13}C$ enriched substrate the subsequently produced $CO_2$ will also be enriched with $^{13}C$.

As mentioned above, the amount of $^{13}C$ enriched $CO_2$ can be measured by conventional methods using Isotope Ratio Mass Spectrometry (IRMS). Therewith, oxidation of a substrate can be followed over time in a non-invasive manner, for both oxidation rate and total oxidation. Practically, this method is a useful screening tool for (fragile) patients who have a metabolic (related) disorder or also for children, for which blood sampling is too invasive and to cumbersome. On a fundamental level, comparison between different substrates can be made, as well as comparison of oxidation rates between different subjects (individuals) and also comparison of active versus non-active subjects is possible. Finally, the results can be correlated to provide the protein status of an individual.

There are several techniques for the preparation of overall $^{13}C$ enriched substrates. One known option for producing $^{13}C$ enriched substrates is given by growing plant material in a $^{13}CO_2$ rich environment, also known as overall high-enrichment labelling. However this option is quite expensive. Another well-known option is to feed animals with plants which are naturally enriched with $^{13}C$, leading to an accordingly $^{13}C$ enriched animal, also known as overall low-enrichment labelling procedure. Suitable $^{13}C$ enrichted fodder plants are naturally occurring so-called $C_4$ plants (or CAM-plants) having a natural $^{13}C$ content of about 1.095%. Examples of $C_4$ plants are maize (corn), sugar cane, sorghum, millet and pineapple.

For comparison, $^{13}C$ poor crops or fodder plants of the group of so-called $C_3$ plants have a natural $^{13}C$ content of about 1.082%. Examples of $C_3$ plants are wheat, rye, oat, rice, grass and hay.

Feeding, for example, cows with corn (from the group of $C_4$ plants) gives $^{13}C$ enriched milk and meat, both being composed of $^{13}C$ enriched proteins, sugars and fat.

The inventors of the present invention found that the method of the invention provides improved results in the fractions of the used milk substrates by using a low-enrichment procedure with feeding corn in addition with cane-sugar or cane-sugar products.

When using a breath test, a suitable $^{13}C$ enriched initial protein substrate is needed. The enriched substrate is administered to an individual, usually in the form of a specific test meal or drink. Then protein oxidation due to protein metabolism can be observed by collecting breath test samples at several pre-determined time points, capturing the individual's exhaled breath by blowing through a kind of straw into a glass tube, container, vial or collection tube with a screwcap on top and a rubber seal in the screwcap so that after thorough exhalation through the straw and thus leading the breath into the glass tube. Finally the tube can be tightly closed and sealed to capture the exhaled breath, thus providing the individual breath test sample. The tubes are then be inserted into the isotope ratio mass spectrometer for analysis.

In principal such breath tests and breath test devices are already known. However, the inventors of the present invention have now found a new method for more precisely determining protein oxidation in such breath test samples.

Therefore, the present invention relates to a new method of quantitatively determining the protein oxidation in an individual, said method comprising the steps of:

a) measuring the $^{13}C$ content in breath test samples of the individual using Isotope Ratio Mass Spectrometry (IRMS),

b) determining the protein oxidation rate [%] per hour from said breath test samples, applying formula (I):

$$\text{recovered amount of } {}^{13}C\ [\%] \text{ per hour} = {}^{13}CO_2 \text{ production } [\%] \times 100 / \text{total initial dose } {}^{13}C$$

with:

$${}^{13}CO_2 \text{ production } [\%] = \text{amount } {}^{13}C \text{ per breath test sample } [\%] \times CO_2 \text{ production} / 100;$$

with

$$CO_2 \text{ production [mmol/hr]} = \text{BSA} \times \varnothing\ CO_2 \text{ mmol / hr / } m^2 \text{ body surface},$$

$$\text{BSA (body surface area) } [m^2] = \text{weight (kg)}^{0.5378} \times \text{length (cm)}^{0.3964} \times 0.024265,$$

and

$$\varnothing\ CO_2 \text{ mmol / hr / } m^2 \text{ body surface} = \text{individual's average } CO_2 \text{ production};$$

and

$$\text{total initial dose } {}^{13}C = (\text{amount of available protein (mg)} \times \text{weighted number of } {}^{13}C \text{ labels} \times (\text{enrichment} - {}^{13}C_{basic} [\%])) / (100 \times \text{weighted molecular weight amino acids } (MW_{testsubstrate}) \text{ [g/mol]});$$

wherein the total initial dose $^{13}C$ is derived from an isolated natural $^{13}C$ low-enriched milk substrate.

In the present invention "∅" relates to the mean value of an individual's average $CO_2$ production.

Preferably, said isolated natural $^{13}C$ low-enriched milk substrate is obtained from cows having been fed with naturally $^{13}C$ enriched maize and sugar cane.

Therein, the breath test samples according to step a) can be obtained, for example, under the conditions as explained in detail in the Examples below. Measurement of the $^{13}C$ content in said breath test samples can be carried out using conventional IRMS methods.

Therefrom, by applying formula (I)

$$\text{recovered amount of } ^{13}C\ [\%] \text{ per hour} = {}^{13}CO_2 \text{ production } [\%] \times 100 / \text{total initial dose } ^{13}C$$

the individual's protein oxidation rate [%] per hour can be determined.

In the formula (I) of the method according to the present invention the individual relative $CO_2$ production [%] at a given point in time is considered, reflected by the value

$$^{13}CO_2 \text{ production } [\%] = \text{amount } ^{13}C \text{ per breath test sample } [\%] \times CO_2 \text{ production} / 100$$

This value reflects the normalized $^{13}CO_2$ production of the individual. This means that from the $^{13}CO_2$ production, determined via the breath test, the $^{13}CO_2$ production naturally occurring in an individual has been eliminated.

Therein, the individual's $CO_2$ production is reflected by the value

$$CO_2 \text{ production [mmol/hr]} = \text{BSA} \times \varnothing\ CO_2 \text{ mmol / hr / m}^2 \text{ body surface}$$

To calculate this value it is necessary to consider how much $CO_2$ the individual will produce (mmol/hr). It is estimated that, for example, an average human individual in rest has a $CO_2$ production of 300 mmol/hr/$m^2$ body surface. Thus, when applying the method of the present invention to human individuals, which is a preferred embodiment of the present invention, the value ∅ $CO_2$ mmol / hr / $m^2$ body surface is 300 mmol / hr / $m^2$ body surface.

Further, for precisely determining the individual protein oxidation the body surface area (BSA) of the individual has to be considered, which can be calculated according to the Haycock formula (Haycock GB, Schwartz GJ, Wisotsky DH "Geometric method for measuring body surface area: A height-weight formula validated in infants, children and adults" J Pediatr 1978, 93:62-66) on the basis of the measurement of the individual's weight and length by the formula

$$\text{BSA} = \text{weight (kg)}^{0.5378} \times \text{length (cm)}^{0.3964} \times 0.024265$$

Accordingly, when the method of the present invention is applied to a human individual, the $CO_2$ production (mmol/hr) of the human individual is BSA x 300.

The determination of the individual relative $CO_2$ production [%] at a given point in time then further reflects the amount $^{13}C$ per breath test sample [%], which is determined from the IRMS measured $^{13}C$ content in the breath test sample.

For determining the individual's protein oxidation rate according to formula (I) of the present invention, it further has to be considered how much $^{13}C$ in total has been offered and thus in principle been made available for the protein digestion process. Then the percentage of the total initial dose $^{13}C$ (mmol) being converted (oxidized) into $^{13}CO_2$ (% dose/hr) in a certain time must be calculated to provide precise values for the protein oxidation. This means, that the total initial dose of $^{13}C$ that has been offered, for example via the initial $^{13}C$ enriched substrate or an $^{13}C$ enriched test meal, must be considered, which can be determined by the formula

$$\text{total initial dose } ^{13}C = (\text{amount of available protein (mg)} \times \text{weighted number of } ^{13}C \text{ labels} \times (\text{enrichment} - {}^{13}C_{basic}\ [\%])) / (100 \times \text{weighted molecular weight amino acids } (MW_{testsubstrate})\ [g/mol])$$

Therein, the term $^{13}C_{basic}$ indicates the basal $^{13}C$ enrichment, which means the naturally occurring $^{13}C$ enrichment at time point zero (t = 0). Every individual contains a certain natural amount of $^{13}C$ isotopes at t = 0. This naturally occurring $^{13}C$ amount has to be subtracted from the $^{13}C$ amount of the enriched substrate to normalize the $^{13}C$ enrichment value.

A further aspect of the present invention relates to a method for determining the cumulated total protein oxidation rate [%] at a time point ($t_x$) by integrating the protein oxidation rate [%] derived from the formula (I) as explained above. The cumulated total protein oxidation rate [%] at a time point ($t_x$) can be derived by applying formula (II)

$$\text{total recovered amount of } ^{13}C \text{ [\%] at } t_x = \text{cumulative } ^{13}C \text{ dose [\%] at } t_{x-1} + ((t_x - t_{x-1}) / 60) \times ((\text{recovered amount } ^{13}C \text{ [\%] per hour at } t_x / 2) + (\text{recovered amount 13C [\%] per hour at } t_{x-1} / 2))$$

Therein the value [recovered amount $^{13}C$ [%] per hour] is determined according to formula (I) as explained above.

This cumulated protein oxidation rate reflects the integration of all the protein oxidation rates at certain time points, excreted in all the very small segments in this period. Thus, the total amount of exhaled $^{13}C$ at a given time can be calculated and displayed as a percentage of the initially offered $^{13}C$ dose.

As mentioned above, the determination of the individual relative $CO_2$ production [%] at a given point in time is calculated on the basis of the measured amount of $^{13}C$ per breath test sample [%]. The presence of $^{13}C$ in a sample is generally given as a $^{13}C$:$^{12}C$ ratio. The $^{13}C$:$^{12}C$ ratio of the breath test samples is then generally compared to the $^{13}C$:$^{12}C$ ratio of an international standard and the difference is noted as 'delta' ($\delta$). As the differences in ratios of the breath test samples and international standard are so small, they are expressed in 'parts per thousand '(‰) deviation from the standard. For carbon this difference is reflected by the formula

$$\delta\, ^{13}C_{sample} = \{(^{13}C{:}^{12}C \text{ ratio}_{sample}) / (^{13}C{:}^{12}C \text{ ratio}_{standard}) - 1\} \times 1000$$

Therein, the standard is defined as 0 ‰. For carbon the international standard is Pee Dee Belemnite (PDB), a carbon formation having the generally accepted absolute $^{13}C$:$^{12}C$ ratio of 0.0112372.

All $^{13}C$ values measured by IRMS are directly returned as the '$\delta$' values, which are then related to the PDB standard according to the formula

$$^{13}C{:}^{12}C \text{ ratio} = ((\delta\, ^{13}C / 1000) + 1) \times 0.0112372$$

Accordingly, in a further aspect of the invention the amount of $^{13}C$ per breath test sample [%], reflected in formula (I) above, can be determined by applying formula (III)

$$\text{amount } ^{13}C \text{ per breath test sample [\%]} = (^{13}C{:}^{12}C \text{ ratio}_{sample} / (^{13}C{:}^{12}C \text{ ratio}_{sample} + 1)) \times 100;$$

with

$$^{13}C{:}^{12}C \text{ ratio}_{sample} = ((\delta\, ^{13}C_{sample} / 1000) + 1) \times \delta\, ^{13}C_{standard},$$

wherein $\delta\, ^{13}C_{standard}$ is the international standard for carbon Pee Dee Belemnite (PDB), having an absolute $^{13}C$:$^{12}C$ ratio of 0.0112372.

The method of the present invention is suitable for determining the protein oxidation in an individual, wherein in the context of the present invention the term "individual" includes any living individual or subject, in particular mammals. Specifically the method of the present invention is suitable for determining the protein oxidation in mammals, which are selected from the group of monogastric mammals, such as omnivores, carnivores as well as herbivores, for example humans, dogs, pigs, rats, cats, horses, and rabbits. Monogastric mammals have a simple single-chambered stomach. In contrast ruminant mammals, like a cow, goat, or sheep, have a complex four-chambered stomach. Very particularly the "individual" is a human individual, although horses (especially race horses) are also preferred individuals. Accordingly, it is particularly preferred to carry out the method of the present invention with breath test samples, which are derived

from a human individual.

Human individuals in the sense of the present invention comprises all kinds of humans such as males, females, elderly people, adults, adolescents, children, babies, newborns, individuals with enhanced physical demands such as in particular athletes, astronauts, individuals with imbalanced or disturbed digestion and nutrition such as for example mal-nourished or undernourished individuals, selectively eating individuals, for example due to diets, vegetarians, vegans, humans suffering from food incompatibilities or intolerances or suffering from food allergies, selectively eating individuals due to physical invalidism or infirmity, selectively eating individuals due to organic mal-functions, organic dysfunctions, e.g. due to deficiencies in the digestive system, due to chemotherapies or any other deficiency related to the digestive system, which may influence the normal digestion, individuals suffering from sarcopenia or being bound to bed (bedrest), individuals with disturbances related to the muscle metabolism etc.. Human individuals of particular interest are elderly people, children, babies, newborns, and individuals with enhanced physical demands such as athletes, astronauts, individuals with imbalanced or disturbed digestion and nutrition, mal-nourished or undernourished individuals, selectively eating individuals, individuals suffering from food incompatibilities or intolerances or suffering from food allergies, individuals suffering from sarcopenia or being bound to bed and individuals with disturbances related to the muscle metabolism.

As mentioned above, the method of the present invention may be particularly suitable for determining an individual protein status. In context therewith, in a further aspect the method of the invention further comprises one or more of the steps

- determining the protein oxidation in an individual under different physical conditions,
- determining the general physical constitution of the individual,
- determining the average physical activity or physical strain of the individual,
- determining the protein status of the individual on the basis of the derived protein oxidation values,
- determining the protein oxidation and protein status of babies for determining the nutritional status,
- choosing suitable nutrition for the individual based on the determined protein status in consideration of his general physical constitution and physical strain, and
- giving nutritional advice for the various categories, in particular for preventing or counteracting under- and malnutrition as well as in particular giving nutritional advice (metabolic programming) for babies for preventing or counteracting obesity and the development of cancer in adult life.

The method of the present invention allows analysis of quantitative differences in metabolic behavior of various proteins in humans, in particular under various physical conditions. The data allow further analysis of macro-nutrient substrate competition in individuals, which can suitably be used in the determination of protein intake recommendations in various metabolic conditions. Finally, the data allow estimation of an individual protein status in various metabolic conditions.

Suitable correlation of the protein oxidation data obtained by the method of the present invention in particular in combination with additional individual data may further allow the estimation of a muscle mass index.

The estimation of a muscle mass index may be particularly suitable, for example, in physical conditions being related with loss of muscle mass, for example during aging (myophathy) leading to immobility, during clinical conditions, due to non-optimal nutrition, during space travel, as well as in physical conditions being related to increase of muscle mass, for example during sport training (e.g. monitoring of increase of performance) or increase of muscle mass in babies (e.g. monitoring of proper development and nutrition).

As mentioned above, the new method of the present invention can particularly suitably be carried out by using breath test samples, wherein the total initial dose $^{13}C$ is derived from an isolated natural $^{13}C$ enriched milk substrate.

Preferably such isolated natural $^{13}C$ enriched milk substrate is selected from at least one of the group consisting of isolated $^{13}C$ labelled total milk protein, isolated $^{13}C$ labelled whey, isolated $^{13}C$ labelled casein, and isolated $^{13}C$ labelled individual milk proteins, or any mixture thereof. Preferably such isolated natural $^{13}C$ enriched milk substrate is a low-enriched milk substrate. More preferably, such isolated natural $^{13}C$ enriched milk substrate is a low-enriched milk substrate, obtained from cows having been fed with naturally $^{13}C$ enriched maize in combination with sugar cane.

Therein the term "total milk protein" means the isolated total proteins of a normal bovine milk, which in average is contained in an amount of approximately 30 to 35 grams of proteins per liter. About 80% of the milk proteins are arranged in casein micelles, which form the largest structures in the fluid portion of the normal milk and which form

aggregates of several thousand protein molecules. Total milk protein in particular comprises the protein sub-fractions casein and whey (approximately 80% casein and 20% whey).

Casein in the sense of the present invention means isolated casein and relates to a kind of sub-fraction of the total milk protein, which comprises several different types of (individual) casein proteins, such as in particular the four main types $\alpha$s1-, $\alpha$s2-, $\beta$-, $\gamma$- and $\kappa$-casein. Collectively, the casein proteins make up around 76 to 86 wt.-% of the total protein content in normal milk. Most of the casein proteins are bound into the casein micelles.

Whey in the sense of the present invention means in particular isolated whey and relates to another kind of sub-fraction of the total milk protein. Whey proteins are more water-soluble than the caseins and do not form larger structures. Because the proteins remain suspended in the whey left behind when the caseins coagulate into curds, they are collectively known as whey proteins. Whey proteins make up approximately 20 wt.-% of the total protein content in milk. Lactoglobulin is the most common whey protein.

Besides, milk contains dozens of other types of proteins beside the caseins and whey proteins, including in particular various enzymes.

Individual milk protein in the sense of the present invention means in particular isolated individual proteins, which are in particular present in the aforementioned sub-fractions casein and whey or in the normal milk.

Examples of individual proteins comprise $\alpha$s1-, $\alpha$s2-, $\beta$-, and $\kappa$-casein, lactoglobulin such as $\beta$-lactoglobulin, lactalbumin, serum albumin and lactoferrin.

Particularly preferred is a method according to the present disclosure wherein the isolated natural $^{13}C$ enriched milk substrate is isolated $^{13}C$ enriched total milk protein, isolated $^{13}C$ enriched whey, isolated $^{13}C$ enriched casein or isolated $^{13}C$ enriched individual milk protein (with individual milk proteins as defined above), which are in the form of a dry powder, preferably a spray-dried powder. Preferably such isolated natural $^{13}C$ enriched milk substrate is a low-enriched milk substrate. More preferably, such isolated natural $^{13}C$ enriched milk substrate is a low-enriched milk substrate, obtained from cows having been fed with naturally $^{13}C$ enriched maize in combination with sugar cane.

Further preferred is a method according to the present disclosure wherein the total initial dose $^{13}C$ is derived from a dose of up to about 70 g, preferably from a dose of up to about 50 g, more preferably from a dose of up to about 30 g of the isolated natural $^{13}C$ enriched milk substrate as defined above.

A further aspect of the present disclosure relates to such a particularly suitable and new isolated natural $^{13}C$ enriched milk substrate, which is isolated natural $^{13}C$ labelled total milk protein, isolated natural $^{13}C$ labelled whey, isolated natural $^{13}C$ labelled casein, or an isolated natural $^{13}C$ labelled individual milk protein, each as defined above, in the form of a dry powder, in particular in the form of a spray-dried powder. Preferably such new isolated natural $^{13}C$ enriched milk substrate is a low-enriched milk substrate. More preferably, such isolated natural $^{13}C$ enriched milk substrate is a low-enriched milk substrate, obtained from cows having been fed with naturally $^{13}C$ enriched maize in combination with sugar cane.

Preferably, the isolated natural $^{13}C$ enriched milk substrate is isolated natural $^{13}C$ labelled total milk protein or isolated natural $^{13}C$ labelled whey, or an isolated natural $^{13}C$ labelled individual milk protein, each as defined above.

Isolation of the respective $^{13}C$ labelled protein fractions or proteins from a $^{13}C$ enriched milk and drying, such as spray-drying, may be carried out by conventional techniques for separating and drying respective (non-labelled) milk fractions.

The new isolated natural $^{13}C$ enriched milk substrate according to the present invention is particularly suitable for carrying out the method of the present invention, as it is characterized by having the following estimated weighted average values per molecule:

| | |
|---|---|
| total milk protein: | weighted average of molecular weight of amino acids of about 131,37 g/mol; weighted average of number of C atoms of amino acids of about 5,13; and $^{13}C$ enrichment of about 1.09561 |
| whey: | weighted average of molecular weight of amino acids of about 130,50 g/mol; weighted average of number of C atoms of amino acids of about 5,01; and $^{13}C$ enrichment of about 1.09561 |
| casein: | weighted average of molecular weight of amino acids of about 131,52 g/mol; weighted average of number of C atoms of amino acids of about 5,15; and $^{13}C$ enrichment of about 1.09561 |

Normally, in isotope studies a well-defined substrate is used, e.g. glucose. Glucose is a molecule with clear values for the weight of the substrate (g/mol) and the number of carbon atoms. For carrying out the method of the present invention a value for weight of the respective initial milk substrate is required as well as the value for the amount of carbon atoms of the milk substrate, for example casein. However, as explained above total milk protein, casein and whey are a collection of different proteins, each having a different amino acid composition and therefore a different mass and different number of carbon atoms. By considering the seven most abundant milk proteins in the respective protein fractions, for example casein, over 90% of the proteins of the respective protein fraction can be accounted for. For these seven most abundant proteins the amino acid content was determined (for example by analysis with known methods

or by calculation from commonly available database values) and for each of said seven proteins a weighted average molecular weight of amino acids and weighted average number of carbon atoms was calculated. On the basis thereof one weighted average molecular weight of amino acids and one weighted average number of carbon atoms was determined as given above.

Particularly preferred is the method according to the present invention, wherein the total initial dose $^{13}C$ is derived from the new isolated natural $^{13}C$ enriched milk substrate as defined above. Preferably, the total initial dose $^{13}C$ is derived from the new isolated natural $^{13}C$ enriched milk substrate which is isolated natural $^{13}C$ labelled total milk protein or isolated natural $^{13}C$ labelled whey, or an isolated natural $^{13}C$ labelled individual milk protein, more preferably said isolated natural $^{13}C$ enriched milk substrate is derived by a low-enrichement method, even more preferred it is obtained from cows having been fed with naturally $^{13}C$ enriched maize in combination with sugar cane.

The invention is illustrated in more detail by the following examples. The examples merely constitute exemplifications, and the person skilled in the art is capable of extending the specific examples to other embodiments for which protection is sought.

DESCRIPTION OF THE FIGURES:

Fig. 1a: Average oxidation kinetics of 30g of total milk protein dissolved in 500ml of water (n=6).

Fig. 1b: Average cumulative oxidation of 30g of total milk protein dissolved in 500ml of water (n=6).

Fig. 2a: Oxidation kinetics of total milk protein given at different dosages

Fig. 2b: Cumulative oxidation of total milk protein given at different dosages

Fig. 3: $^{13}CO_2$ oxidation kinetics of 2 substrates; red line: whey (fast) and green line: casein (slow)

Fig. 4: Different substrates result in different oxidation kinetics

Fig. 5a: $^{13}CO_2$ oxidation kinetics of total milk protein under 3 different circumstances; blue line: 30g total milk protein during rest, grey line: 30g total milk protein during rest after 3 days of protein restriction diet (~10g/day) prior to testing, orange line: 30g total milk protein during rest after 3 days of protein restriction diet (~10g/day) prior to testing plus 1 hour, 3 km swim on the morning prior to the test

Fig. 5b: $^{13}CO_2$ oxidation cumulative of total milk protein under 3 different circumstances; blue line: 30g total milk protein during rest, grey line: 30g total milk protein during rest after 3 days of protein restriction diet (~10g/day) prior to testing, orange line: 30g total milk protein during rest after 3 days of protein restriction diet (~10g/day) prior to testing plus 1 hour, 3 km swim in the morning prior to the test

Fig. 6: Relative endpoint (t=330) comparison of 30g total milk protein oxidation under different circumstances; blue (100%): total milk protein oxidation during rest, orange: 30g total milk protein during rest after 3 days of protein restriction diet (~10g/day) prior to testing, yellow: 30g total milk protein during rest after 30 minutes of cycling (~130 bpm, 135 watt, 80 rpms) in the morning prior to the test, grey: 30g total milk protein during rest after 3 days of protein restriction diet (~10g/day) prior to testing plus 1 hour, 3 km swim in the morning prior to the test

EXAMPLES:

**I. Milk Substrate**

The $^{13}C$ enriched milk substrate was prepared by restricting the food of cows to a combination of maize and sugar cane to obtain naturally $^{13}C$ labelled milk, having a $\delta$ $^{13}C$ value of 14.21. The $^{13}C$ labelled (whole) milk was separated into 5 different milk fractions such as total milk protein, whey, casein, lactose and milk fat. The total milk protein, whey, casein and lactose fraction were dried using spray drying and provided as a powder of isolated $^{13}C$ labelled total milk protein, isolated $^{13}C$ labelled whey, isolated $^{13}C$ labelled casein and isolated $^{13}C$ labelled lactose.

**II. Breath Test**

II.1. Preparation of the initial $^{13}C$ enriched substrate ($^{13}C$ enriched test meal)

The respective $^{13}C$ labelled milk substrate powder is dissolved in water. A dose of 30 g of the milk substrate powder is dissolved in 500ml of water.

11.2. Breath Test

2.1 Day(s) before the breath test

The breath test was carried out with adult human volunteers.

In order to keep the $^{13}C$-level in each test subject as low as possible, consumption of naturally enriched $^{13}C$ products should be eliminated two days before the test.

To minimize an influence of exercise/sports on the fate of the test substrate, whether or not the given substrate is oxidized or stored, exercise/sports should be avoided 1 day before the test and during the test day.

To arrive sober at the day of testing consumption of food and drinks should be stopped from 22:00 the night before the test. Also breakfast on the day of the test should be avoided. Drinking water or coffee without sugar and/or milk was allowed.

2.2 Day of testing

Basal breath samples are taken a few minutes before the consumption of the initial test meal (i.e. in the form of a test drink of 30 gram of the desired $^{13}C$ labelled test substrate + 500ml of water to dilute the test substrate). The test drink is consumed at 09:15 within 5 minutes and breath samples are taken from 09:25 every 10 minutes until 14:45. If a test subject was not able to postpone eating or drinking until 14:45 a note of the time point and the type of consumption was made.

Every deviation in the timing of sampling compared to the schedule is noted.

2.3 Sampling

Breath samples are collected in so called breath containers. These breath containers consist of a glass tube with a top screwcap having a rubber sealing.

The test subject opens the container and inserts a straw into the glass container nearly all the way to the bottom. The subject takes a good breath and blows out via the straw into the breath container by blowing out deeply. Immediately after exhalation, the cap is screwed back on. The longer it takes to screw the cap, the more expelled air will be lost from the sampling container.

The breath samples are ideally measured as soon as possible, however the samples have a shelf-life of at least 4 weeks.

2.4 IRMS Measurement

The $^{13}C$ amount in the collected breath samples is measured using conventional IRMS methods and devices.

**III. Results**

From the measured IRMS-derived delta $^{13}C$ values the protein oxidation rate and the cumulative protein oxidation was determined in accordance with the present invention.

111.1 Oxidation of milk proteins: kinetics and cumulative

Figure 1a shows the average oxidation kinetics of 30g of total milk protein dissolved in 500ml of water over 330 minutes (6 repeats within 1 subject). Maximal oxidation (± standard deviation SD) is reached at 120 min after ingestion (7.24% (±1.54%)). Ascent is faster than descent. At 330 minutes oxidation has not returned to baseline levels.

Figure 1b shows the cumulative oxidation of 30g of total milk protein dissolved in 500ml of water over 330 minutes (6 repeats). At endpoint (t=330 minutes) 24.18% (±3,37%) of the given dose is recovered as $^{13}CO_2$ and is thus oxidized.

III.2 Different doses of total milk proteins

Figure 2a shows the oxidation kinetic results of different dosages of total milk protein ranging from 10g to 70g. The 10 to 50g curves are comparable, however the 70g dosage results in a more slowly ascending part with flows into a plateau rather than a clear peak.

Figure 2b shows the cumulative oxidation of total milk protein. At endpoint (330 minutes) the amount oxidized is similar for 10, 30 and 50g doses. The 70g dose shows a lower oxidation value.

III.3 Oxidation profiles of whey and casein (Breath analyses vs blood analyses)

Boirie et al. 1997 measured the appearance of $^{13}$C-whey and $^{13}$C-casein in blood via $^{13}$C labelled leucine. These results served as a template for what to expect when measuring the appearance of $^{13}$C in $^{13}CO_2$ from exhaled breath. Boirie et al have found that whey appears quickly compared to casein and has a clear peak in oxidation rate. Casein however forms a plateau quickly and after 5 hours starts to diminish slowly compared to the rate of decrease seen in the signal from whey. Based on these observations proteins are classified as "slow" or "fast" proteins.

As shown in figure 3, whey and casein were given on separate occasions. Each substrate was given as a 30 g dose diluted in 500 ml of water. Based on the findings of Boirie et al 1997, whey should yield a curve with fast increase in oxidation rate ending in a peak after which the oxidation rate should diminish. In comparison, casein should yield a curve with slow increase in oxidation rate following into a plateau which slowly decreases.

III.4 Oxidation of different substrates (Whey, casein, milk fat and lactose)

Figure 4 shows the results of different substrates: whey, casein, total milk protein, milk fat and lactose. Strikingly, whey is oxidized faster than lactose. A clear difference in kinetics is seen between whey and casein, where whey peaks fast and then decreases all the way close to baseline. Casein however ascends slowly, forms a plateau and slowly decreases towards baseline. Total milk protein which consists of 80% casein and 20% whey behaves mostly as casein, although total milk protein has a slightly higher peak than casein. Milk fat is most slowly oxidized, a flattened peak is reached between 4 and 5 hours.

At endpoint (330 minutes) the amount oxidized is the same for whey and lactose (30 to 35%). Also casein, total milk protein and milk fat are grouped together at endpoint (22 to 26%).

111.5 Normal diet vs protein restricted diet

Figure 5a shows the oxidation kinetics of 30g total milk protein under different circumstances. In blue is the oxidation under normal resting conditions. In grey is the oxidation with 3 days prior to the experiment a protein restriction diet (~10g/day). The orange oxidation line also has 3 days of protein restriction plus on the morning before the test a 1 hour, 3km swim. The oxidation kinetics clearly change with increasing severity of intervention. The ascending part of all lines is similar, but the descending part is different, with the orange line even going through baseline level.

Figure 5b shows the cumulative curves with clear separation from time point 120 minutes. Total milk protein without any intervention shows the highest oxidation at the endpoint, followed by total milk protein with protein restriction (3 days prior to testing, ~10/day) and with lowest oxidation is total milk protein with protein restriction plus exercise (1 hour, 3 km swim).

111.6 Relative endpoint comparison

Figure 6 shows the relative endpoints of total milk protein with different interventions. Compared to no intervention (blue) there is a ~20% reduction when a protein restriction diet (3 days, ~10g/day) is added to the protocol (orange). A ~40% reduction (yellow) compared to control is seen with cycling (~130 bpm, 135 watt, 80 rpms), however a large standard deviation can be noted. ~50% reduction is seen when protein restriction is combined with exercise (1 hour, 3 km swim) just prior to testing.

## Claims

1. A method of quantitatively determining the protein oxidation in an individual, said method comprising the steps of:

a) measuring the $^{13}$C content in breath test samples of the individual using Isotope Ratio Mass Spectrometry (IRMS),
b) determining the protein oxidation rate [%] per hour from said breath test samples, applying formula (I):

$$\text{recovered amount of } ^{13}\text{C [\%] per hour} = {}^{13}\text{CO}_2 \text{ production [\%] x 100 / total initial dose } ^{13}\text{C}$$

with:

$$^{13}CO_2 \text{ production [\%]} = \text{amount } ^{13}C \text{ per breath test sample [\%]} \times CO_2 \text{ production [mmol / hr] / 100;}$$

$$CO_2 \text{ production [mmol/hr]} = \text{BSA} \times \varnothing\, CO_2 \text{ mmol / hr / } m^2 \text{ body surface,}$$

$$\text{BSA (body surface area) } [m^2] = \text{weight (kg)}^{0.5378} \times \text{length (cm)}^{0.3964} \times 0.024265,$$

$$\varnothing\, CO_2 \text{ mmol / hr / } m^2 \text{ body surface} = \text{individual's average } CO_2 \text{ production [mmol / hr / } m^2 \text{ body surface]}$$

and

$$\text{total initial dose } ^{13}C = (\text{amount of available protein (mg)} \times \text{weighted number of } ^{13}C \text{ labels} \times (\text{enrichment} - {}^{13}C_{basic} [\%])) / (100 \times \text{weighted molecular weight amino acids } (MW_{testsubstrate}) \text{ [g/mol]});$$

wherein the total initial dose $^{13}C$ is derived from an isolated naturally $^{13}C$ enriched milk substrate which is selected from isolated $^{13}C$ labelled total milk protein, isolated $^{13}C$ labelled whey and isolated $^{13}C$ labelled individual milk proteins which are selected from the group consisting of lactoglobulin, lactalbumin, serum albumin and lactoferrin, preferably the isolated naturally $^{13}C$ enriched milk substrate is isolated $^{13}C$ labelled whey.

**2.** The method according to claim 1, further comprising step
c) determining the cumulated total protein oxidation rate [%] at a time point ($t_x$) by integrating the protein oxidation rate according to claim 1, applying formula (II):

$$\text{total recovered amount of } ^{13}C \text{ [\%] at } t_x = \text{cumulative } ^{13}C \text{ dose [\%] at } t_{x-1} + ((t_x - t_{x-1}) / 60) \times ((\text{recovered amount } ^{13}C \text{ [\%] per hour at } t_x / 2) + (\text{recovered amount } ^{13}C \text{ [\%] per hour at } t_{x-1} / 2));$$

wherein the value [recovered amount $^{13}C$ [%] per hour] is determined according to formula (I) of claim 1.

**3.** The method according to any one of the preceding claims, wherein the value [amount $^{13}C$ per breath test sample [%]] derives from the formula (III):

$$\text{amount } ^{13}C \text{ per breath test sample [\%]} = (^{13}C{:}^{12}C \text{ ratio}_{sample} / (^{13}C{:}^{12}C \text{ ratio}_{sample} + 1)) \times 100;$$

with

$$^{13}C{:}^{12}C \text{ ratio}_{sample} = ((\delta\, ^{13}C_{sample} / 1000) + 1) \times \delta\, ^{13}C_{standard},$$

wherein $\delta\, ^{13}C_{standard}$ is the international standard for carbon Pee Dee Belemnite (PDB), having an absolute $^{13}C{:}^{12}C$ ratio of 0.0112372.

**4.** The method according to any one of the preceding claims, wherein the total initial dose $^{13}C$ is derived from an isolated naturally $^{13}C$ enriched milk substrate selected from isolated $^{13}C$ labelled total milk protein, isolated $^{13}C$ labelled whey and isolated $^{13}C$ labelled individual milk proteins which are selected from the group consisting of lactoglobulin, lactalbumin, serum albumin and lactoferrin, preferably the isolated naturally $^{13}C$ enriched milk substrate is isolated $^{13}C$ labelled whey, wherein the isolated naturally $^{13}C$ enriched milk substrate is obtained from cows having

been fed with naturally $^{13}C$ enriched maize and sugar cane.

5. The method according to any one of the preceding claims, wherein the isolated naturally $^{13}C$ enriched milk substrate selected from isolated $^{13}C$ labelled total milk protein, isolated $^{13}C$ labelled whey, and isolated $^{13}C$ labelled individual milk protein which are selected from the group consisting of lactoglobulin, lactalbumin, serum albumin and lactoferrin, preferably being isolated $^{13}C$ labelled whey, is in the form of a dry powder.

6. The method according to any one of the preceding claims, wherein the isolated naturally $^{13}C$ enriched milk substrate is total milk protein or whey , having the following estimated weighted average values per molecule:

| | |
|---|---|
| total milk protein: | weighted average of molecular weight of amino acids of about 131,37 g/mol; weighted average of number of C atoms of amino acids of about 5,13; and $^{13}C$ enrichment of about 1.09561 |
| whey: | weighted average of molecular weight of amino acids of about 130,50 g/mol; weighted average of number of C atoms of amino acids of about 5,01; and $^{13}C$ enrichment of about 1.09561. |

7. The method according to any one of the preceding claims, wherein the total initial dose $^{13}C$ is derived from a dose of up to 30 g of the isolated naturally $^{13}C$ enriched milk substrate.

8. An isolated naturally $^{13}C$ enriched milk substrate, selected from isolated $^{13}C$ labelled total milk protein, isolated $^{13}C$ labelled whey and isolated $^{13}C$ labelled individual milk proteins which are selected from the group consisting of lactoglobulin, lactalbumin, serum albumin and lactoferrin, preferably isolated $^{13}C$ labelled whey, which is in the form of a dry powder.

9. The isolated naturally $^{13}C$ enriched milk substrate according to claim 8, which is obtained from cows having been fed with naturally $^{13}C$ enriched maize and sugar cane.

10. The isolated naturally $^{13}C$ enriched milk substrate according to claim 8 or 9, which is total milk protein or whey , having the following estimated weighted average values per molecule:

| | |
|---|---|
| total milk protein: | weighted average of molecular weight of amino acids of about 131,37 g/mol; weighted average of number of C atoms of amino acids of about 5,13; and $^{13}C$ enrichment of about 1.09561 |
| whey: | weighted average of molecular weight of amino acids of about 130,50 g/mol; weighted average of number of C atoms of amino acids of about 5,01; and $^{13}C$ enrichment of about 1.09561. |

11. The method according to any one of claims 1 to 7, wherein the breath test samples are derived from a human individual.

12. The method according to claim 11, wherein the human individual is selected from the group consisting of elderly people, adolescents, children, babies, newborns, and individuals with enhanced physical demands such as athletes, astronauts, individuals with imbalanced or disturbed digestion and nutrition, mal-nourished or undernourished individuals, selectively eating individuals, individuals suffering from food incompatibilities or intolerances or suffering from food allergies, individuals suffering from sarcopenia or being bound to bed, individuals with disturbances related to the muscle metabolism.

13. The method according to claim 11 or 12, wherein the value [∅ $CO_2$ mmol / hr / $m^2$ body surface] is 300 mmol / hr / $m^2$ body surface.

14. The method according to any one of claims 1 to 7 and 11 to 13, further comprising one or more of the steps

- determining the protein oxidation in an individual under different physical conditions,
- determining the general physical constitution of the individual,
- determining the average physical activity or physical strain of the individual,
- determining the protein status of the individual on the basis of the derived protein oxidation values,
- determining the protein oxidation and protein status of babies for determining the nutritional status,

- choosing a suitable nutrition for the individual based on the determined protein status in consideration of his general physical constitution and physical strain,
- giving nutritional advices for the various categories, in particular for preventing or counteracting undernutrition or giving nutritional advice for babies for preventing or counteracting obesity and the development of cancer in adult life.

**15.** The method according to any one of claims 1 to 7 and 11 to 14, wherein the total initial dose $^{13}C$ is derived from an isolated naturally $^{13}C$ enriched milk substrate according to claim 8, 9 or 10.

**Patentansprüche**

**1.** Verfahren zur quantitativen Bestimmung der Proteinoxidation in einem Individuum, wobei das Verfahren die folgenden Schritte umfasst:

a) Messen des $^{13}C$-Gehaltes in Atemtestproben des Individuums mittels Isotopenverhältnis-Massenspektrometrie (IRMS),
b) Bestimmen der Proteinoxidationsrate [%] pro Stunde aus den Atemtestproben unter Anwendung der Formel (I):

$$\text{wiedergewonnene Menge an } ^{13}C\ [\%] \text{ pro Stunde} = {}^{13}CO_2\text{-Produktion } [\%] \times 100 / {}^{13}C\text{-Gesamtanfangsdosis}$$

mit:

$$^{13}CO_2\text{-Produktion } [\%] = \text{Menge } ^{13}C \text{ pro Atemtestprobe } [\%] \text{ x } CO_2\text{-Produktion } [mmol / h] / 100;$$

$$CO_2\text{-Produktion } [mmol / h] = \text{BSA x } \varnothing\ CO_2 \text{ mmol / h / } m^2 \text{ Körperoberfläche},$$

$$\text{BSA (Fläche der Körperoberfläche) } [m^2] = \text{Gewicht (kg)}^{0.5378} \text{ x Länge (cm)}^{0.3964} \text{ x } 0.024265,$$

$$\varnothing\ CO_2 \text{ mmol / h / } m^2 \text{ Körperoberfläche} = \text{durchschnittliche } CO_2\text{-Produktion des Individuums } [mmol / h / m^2 \text{ Körperoberfläche}]$$

und

$$^{13}C\text{-Gesamtanfangsdosis} = (\text{Menge verfügbaren Proteins (mg) x gewichtete Anzahl von } ^{13}C\text{-Markierungen x (Anreicherung - } ^{13}C_{basis} [\%])) / (100 \text{ x gewichtetes Molekulargewicht der Aminosäuren } (MW_{Testsubstrat}) [g/mol]);$$

worin die $^{13}C$-Gesamtanfangsdosis von einem isolierten natürlich $^{13}C$-angereicherten Milchsubstrat stammt, welches ausgewählt ist aus isoliertem $^{13}C$-markiertem Gesamtmilchprotein, isolierter $^{13}C$-markierter Molke, isolierten $^{13}C$-markierten einzelnen Milchproteinen die ausgewählt sind aus der Gruppe bestehend aus Lactoglobulin, Lactalbumin, Serumalbumin und Lactoferrin, bevorzugt ist das isolierte natürlich $^{13}C$-angereicherte Milchsubstrat isolierte $^{13}C$-markierte Molke.

**2.** Verfahren gemäß Anspruch 1, ferner umfassend Schritt
c) Bestimmen der kumulierten Gesamtproteinoxidationsrate [%] zu einem Zeitpunkt ($t_x$) durch Integrieren der Proteinoxidationsrate nach Anspruch 1, Anwenden der Formel (II):

Gesamtmenge des wiedergewonnenen $^{13}C$ [%] bei $t_x$ = kumulative $^{13}C$-Dosis [%] bei $t_{x-1}$ + $((t_x - t_{x-1}) / 60)$ x ((wiedergewonnene Menge an $^{13}C$ [%] pro Stunde bei $t_x$ / 2) + (wiedergewonnene Menge an $^{13}C$ [%] pro Stunde bei $t_{x-1}$ / 2));

worin der Wert [wiedergewonnene Menge an $^{13}C$ [%] pro Stunde] gemäß Formel (I) aus Anspruch 1 bestimmt wird.

3. Verfahren gemäß einem der vorhergehenden Ansprüche, worin der Wert [$^{13}C$-Menge pro Atemtestprobe [%]] aus der Formel (III) stammt:

$$^{13}C\text{-Menge pro Atemtestprobe [\%]} = (^{13}C{:}^{12}C\ \text{Verhältnis}_{Probe} / (^{13}C{:}^{12}C\ \text{Verhältnis}_{Probe} + 1)) \times 100;$$

mit

$$^{13}C{:}^{12}C\ \text{Verhältnis}_{Probe} = ((\delta\ ^{13}C_{Probe} / 1000) + 1) \times \delta\ ^{13}C_{Standard},$$

worin $\delta\ ^{13}C_{Standard}$ der internationale Standard für Kohlenstoff Pee Dee Belemnit (PDB) ist, mit einem absoluten $^{13}C$:$^{12}C$ Verhältnis von 0.0112372.

4. Verfahren gemäß einem der vorhergehenden Ansprüche, worin die $^{13}C$-Gesamtanfangsdosis von einem isolierten natürlich $^{13}C$-angereicherten Milchsubstrat stammt, welches ausgewählt ist aus isoliertem $^{13}C$-markiertem Gesamtmilchprotein, isolierter $^{13}C$-markierter Molke, isolierten $^{13}C$-markierten einzelnen Milchproteinen die ausgewählt sind aus der Gruppe bestehend aus Lactoglobulin, Lactalbumin, Serumalbumin und Lactoferrin, bevorzugt ist das isolierte natürlich $^{13}C$-angereicherte Milchsubstrat isolierte $^{13}C$-markierte Molke, wobei das isolierte natürlich $^{13}C$-angereicherte Milchsubstrat von Kühen erhalten wird, die mit natürlich $^{13}C$-angereichertem Mais und Rohrzucker gefüttert wurden.

5. Verfahren gemäß einem der vorhergehenden Ansprüche, worin das isolierte natürlich $^{13}C$-angereicherte Milchsubstrat ausgewählt aus isoliertem $^{13}C$-markiertem Gesamtmilchprotein, isolierter $^{13}C$-markierter Molke, isolierten $^{13}C$-markierten einzelnen Milchproteinen die ausgewählt sind aus der Gruppe bestehend aus Lactoglobulin, Lactalbumin, Serumalbumin und Lactoferrin in Form eines trockenen Pulvers vorliegt.

6. Verfahren gemäß einem der vorhergehenden Ansprüche, worin das isolierte natürlich $^{13}C$-angereicherte Milchsubstrat Gesamtmilchprotein oder Molke ist mit den folgenden erwarteten gewichteten durchschnittlichen Werten pro Molekül:

| | |
|---|---|
| Gesamtmilchprotein: | gewichtetes durchschnittliches Molekulargewicht der Aminosäuren von etwa 131,37 g/mol; gewichtete durchschnittliche Anzahl von C-Atomen der Aminosäuren von etwa 5,13; und $^{13}C$ Anreicherung von etwa 1.09561 |
| Molke: | gewichtetes durchschnittliches Molekulargewicht der Aminosäuren von etwa 130,50 g/mol; gewichtete durchschnittliche Anzahl von C-Atomen der Aminosäuren von etwa 5,01; und $^{13}C$ Anreicherung von etwa 1.09561. |

7. Verfahren gemäß einem der vorhergehenden Ansprüche, worin die $^{13}C$-Gesamtanfangsdosis von einer Dosis von bis zu 30 g isoliertem natürlich $^{13}C$-angereichertem Milchsubstrat stammt.

8. Isoliertes natürlich $^{13}C$-angereichertes Milchsubstrat ausgewählt aus isoliertem $^{13}C$-markiertem Gesamtmilchprotein, isolierter $^{13}C$-markierter Molke, isolierten $^{13}C$-markierten einzelnen Milchproteinen die ausgewählt sind aus der Gruppe bestehend aus Lactoglobulin, Lactalbumin, Serumalbumin und Lactoferrin, bevorzugt isolierte $^{13}C$-markierte Molke, in Form eines trockenen Pulvers.

9. Isoliertes natürlich $^{13}C$-angereichertes Milchsubstrat gemäß Anspruch 8, welches von Kühen erhalten wird, die mit

natürlich $^{13}C$-angereichertem Mais und Rohrzucker gefüttert wurden.

**10.** Isoliertes natürlich $^{13}C$-angereichertes Milchsubstrat gemäß Anspruch 8 oder 9, welches Gesamtmilchprotein oder Molke ist, mit den folgenden erwarteten gewichteten durchschnittlichen Werten pro Molekül:

| | |
|---|---|
| Gesamtmilchprotein: | gewichtetes durchschnittliches Molekulargewicht der Aminosäuren von etwa 131,37 g/mol; gewichtete durchschnittliche Anzahl von C-Atomen der Aminosäuren von etwa 5,13; und $^{13}C$ Anreicherung von etwa 1.09561 |
| Molke: | gewichtetes durchschnittliches Molekulargewicht der Aminosäuren von etwa 130,50 g/mol; gewichtete durchschnittliche Anzahl von C-Atomen der Aminosäuren von etwa 5,01; und $^{13}C$ Anreicherung von etwa 1.09561. |

**11.** Verfahren gemäß einem der Ansprüche 1 bis 7, worin die Atemtestproben von einem menschlichen Individuum stammen.

**12.** Verfahren gemäß Anspruch 11, worin das menschliche Individuum ausgewählt ist aus der Gruppe bestehend aus älteren Menschen, Jugendlichen, Kindern, Babys, Neugeborenen und Individuen mit erhöhten körperlichen Anforderungen, wie Sportlern, Astronauten, Individuen mit unausgewogener oder gestörter Verdauung und Ernährung, mangelernährten oder unterernährten Individuen, selektiv-essenden Individuen, Individuen die an Nahrungsmittelunverträglichkeiten oder -intoleranzen oder an Nahrungsmittelallergien leiden, Individuen die an Sarkopenie leiden oder die ans Bett gefesselt sind, Individuen mit Störungen im Muskelstoffwechsel.

**13.** Verfahren gemäß Anspruch 11 oder 12, worin der Wert [$\varnothing$ $CO_2$ mmol / h / $m^2$ Körperoberfläche] 300 mmol / h / $m^2$ Körperoberfläche beträgt.

**14.** Verfahren gemäß einem der Ansprüche 1 bis 7 und 11 bis 13, ferner umfassend einen oder mehrere der Schritte

- Bestimmen der Proteinoxidation in einem Individuum unter verschiedenen physischen Bedingungen,
- Bestimmen der allgemeinen physischen Konstitution des Individuums,
- Bestimmen der durchschnittlichen physischen Aktivität oder physischen Belastung des Individuums,
- Bestimmen des Proteinstatus des Individuums auf der Grundlage der abgeleiteten Proteinoxidationswerte,
- Bestimmen der Proteinoxidation und des Proteinstatus von Babys zur Bestimmung des Ernährungsstatus,
- Auswahl einer geeigneten Ernährung für das Individuum auf der Grundlage des ermittelten Proteinstatus unter Berücksichtigung seiner allgemeinen physischen Verfassung und physischen Belastung,
- Ernährungsempfehlungen für die verschiedenen Kategorien, insbesondere zur Verhinderung oder Bekämpfung von Unterernährung oder Ernährungsberatung für Babys zur Verhinderung oder Bekämpfung von Adipositas und der Entwicklung von Krebs im Erwachsenenalter.

**15.** Verfahren nach einem der Ansprüche 1 bis 7 und 11 bis 14, worin die $^{13}C$-Gesamtanfangsdosis von einem isolierten natürlich $^{13}C$-angereichertem Milchsubstrat gemäß Anspruch 8, 9 oder 10 stammt.

## Revendications

**1.** Procédé de détermination quantitative de l'oxydation de protéines chez un individu, ledit procédé comprenant les étapes qui consistent :

a) à mesurer la teneur en $^{13}C$ dans des échantillons de test respiratoire de l'individu en utilisant la Spectrométrie de Masse de Rapport Isotopique (IRMS),
b) à déterminer le taux d'oxydation de protéines [%] par heure à partir desdits échantillons de test respiratoire, en appliquant la formule (I) :

$$\text{quantité récupérée de } ^{13}C\ [\%] \text{ par heure} = \text{production de } ^{13}CO_2\ [\%] \times 100/\text{dose initiale totale de } ^{13}C$$

avec :

production de $^{13}CO_2$ [%] = quantité de $^{13}C$ par échantillon de test respiratoire [%] x production de $CO_2$ [mmol/h]/100 ;

production de $CO_2$ [mmol/h] = BSA x Ø $CO_2$ mmol/h/m² de surface corporelle,

BSA (surface corporelle) [m²] = poids $(kg)^{0,5378}$ x longueur $(cm)^{0,3964}$ x 0,024265,

Ø $CO_2$ mmol/h/m² de surface corporelle = production moyenne de $CO_2$ de l'individu [mmol/h/m² de surface corporelle]

et

dose initiale totale de $^{13}C$ = (quantité de protéine disponible (mg) x nombre pondéré de marqueurs $^{13}C$ x (enrichissement - $^{13}C_{basique}$ [%]))/(100 x poids moléculaire pondéré d'acides aminés ($PM_{substrattest}$) [g/mol]) ;

où la dose initiale totale de $^{13}C$ provient d'un substrat laitier naturellement enrichi en $^{13}C$ isolé, qui est choisi parmi les protéines totales de lait marquées au $^{13}C$ isolées, un lactosérum marqué au $^{13}C$ isolé et des protéines de lait individuelles marquées au $^{13}C$ isolées qui sont choisies dans le groupe constitué par la lactoglobuline, la lactalbumine, l'albumine sérique et la lactoferrine, de préférence le substrat laitier naturellement enrichi en $^{13}C$ isolé est du lactosérum marqué au $^{13}C$ isolé.

**2.** Procédé selon la revendication 1, comprenant en outre l'étape qui consiste
c) à déterminer le taux d'oxydation de protéines totales cumulé [%] à un instant ($t_x$) par intégration du taux d'oxydation de protéines selon la revendication 1, en appliquant la formule (II) :

quantité récupérée totale de $^{13}C$ [%] à $t_x$ = dose cumulée de $^{13}C$ [%] à $t_{x-1}$ + (($t_x$-$t_{x-1}$)/60) x ((quantité récupérée de $^{13}C$ [%] par heure à $t_x$/2) + (quantité récupérée de $^{13}C$ par heure à $t_{x-1}$/2)) ;

où la valeur [quantité récupérée de $^{13}C$ [%] par heure] est déterminée selon la formule (I) de la revendication 1.

**3.** Procédé selon l'une quelconque des revendications précédentes, dans lequel la valeur [quantité de $^{13}C$ par échantillon de test respiratoire [%]] provient de la formule (III) :

quantité de $^{13}C$ par échantillon de test respiratoire [%] = ($rapport_{échantillon}$ $^{13}C$ : $^{12}C$/($rapport_{échantillon}$ $^{13}C$ : $^{12}C$ + 1)) x 100 ;

avec

$$\text{rapport}_{\text{échantillon}}{}^{13}C : {}^{12}C = ((\delta\ {}^{13}C_{\text{échantillon}}/1000)+1) \times \delta\ {}^{13}C_{\text{standard}},$$

où $\delta\ ^{13}C_{standard}$ est la norme internationale pour le carbone Pee Dee Belemnite (PDB) ayant un rapport $^{13}C : {}^{12}C$ absolu de 0,0112372.

4. Procédé selon l'une quelconque des revendications précédentes, dans lequel la dose initiale totale de $^{13}C$ provient d'un substrat laitier naturellement enrichi en $^{13}C$ isolé choisi parmi les protéines totales de lait marquées au $^{13}C$ isolées, un lactosérum marqué au $^{13}C$ isolé et des protéines de lait individuelles marquées au $^{13}C$ isolées qui sont choisies dans le groupe constitué par la lactoglobuline, la lactalbumine, l'albumine sérique et la lactoferrine, de préférence le substrat laitier naturellement enrichi en $^{13}C$ isolé est du lactosérum marqué au $^{13}C$ isolé, où le substrat laitier naturellement enrichi en $^{13}C$ isolé est obtenu à partir de vaches ayant été nourries avec du maïs et de la canne à sucre naturellement enrichis en $^{13}C$.

5. Procédé selon l'une quelconque des revendications précédentes, dans lequel le substrat laitier naturellement enrichi en $^{13}C$ isolé choisi parmi les protéines totales de lait marquées au $^{13}C$ isolées, un lactosérum marqué au $^{13}C$ isolé et des protéines de lait individuelles marquées au $^{13}C$ isolées qui sont choisies dans le groupe constitué par la lactoglobuline, la lactalbumine, l'albumine sérique et la lactoferrine, de préférence qui est du lactosérum marqué au $^{13}C$ isolé, se présente sous la forme d'une poudre sèche.

6. Procédé selon l'une quelconque des revendications précédentes, dans lequel le substrat laitier naturellement enrichi en $^{13}C$ isolé représente des protéines totales de lait ou du lactosérum, ayant les valeurs moyennes pondérées estimées suivantes par molécule :

| | |
|---|---|
| protéines totales de lait : | moyenne pondérée du poids moléculaire d'acides aminés d'environ 131,37 g/mol ; moyenne pondérée du nombre d'atomes de C d'acides aminés d'environ 5,13 ; et enrichissement en $^{13}C$ d'environ 1,09561 |
| lactosérum : | moyenne pondérée du poids moléculaire d'acides aminés d'environ 130,50 g/mol ; moyenne pondérée du nombre d'atomes de C d'acides aminés d'environ 5,01 ; et enrichissement en $^{13}C$ d'environ 1,09561. |

7. Procédé selon l'une quelconque des revendications précédentes, dans lequel la dose initiale totale de $^{13}C$ provient d'une dose allant jusqu'à 30 g du substrat laitier naturellement enrichi en $^{13}C$ isolé.

8. Substrat laitier naturellement enrichi en $^{13}C$ isolé, choisi parmi les protéines totales de lait marquées au $^{13}C$ isolées, un lactosérum marqué au $^{13}C$ isolé et des protéines de lait individuelles marquées au $^{13}C$ isolées qui sont choisies dans le groupe constitué par la lactoglobuline, la lactalbumine, l'albumine sérique et la lactoferrine, de préférence du lactosérum marqué au $^{13}C$ isolé, qui se présente sous la forme d'une poudre sèche.

9. Substrat laitier naturellement enrichi en $^{13}C$ isolé selon la revendication 8, qui est obtenu à partir de vaches ayant été nourries avec du maïs et de la canne à sucre naturellement enrichis en $^{13}C$.

10. Substrat laitier naturellement enrichi en $^{13}C$ isolé selon la revendication 8 ou 9, qui représente des protéines totales de lait ou du lactosérum, ayant les valeurs moyennes pondérées estimées suivantes par molécule :

| | |
|---|---|
| protéines totales de lait : | moyenne pondérée du poids moléculaire d'acides aminés d'environ 131,37 g/mol ; moyenne pondérée du nombre d'atomes de C d'acides aminés d'environ 5,13 ; et enrichissement en $^{13}C$ d'environ 1,09561 |
| lactosérum : | moyenne pondérée du poids moléculaire d'acides aminés d'environ 130,50 g/mol ; moyenne pondérée du nombre d'atomes de C d'acides aminés d'environ 5,01 ; et enrichissement en $^{13}C$ d'environ 1,09561. |

11. Procédé selon l'une quelconque des revendications 1 à 7, dans lequel les échantillons de test respiratoire sont dérivés d'un individu humain.

**12.** Procédé selon la revendication 11, dans lequel l'individu humain est choisi dans le groupe constitué par des personnes âgées, des adolescents, des enfants, des bébés, des nouveau-nés et des individus ayant des exigences physiques accrues tels que les athlètes, les astronautes, des individus ayant une digestion et une nutrition déséquilibrées ou perturbées, des individus souffrant de malnutrition ou de dénutrition, des individus mangeant sélectivement, des individus souffrant d'incompatibilités ou d'intolérances alimentaires ou souffrant d'allergies alimentaires, des individus souffrant de sarcopénie ou alités, des individus souffrant de troubles liés au métabolisme musculaire.

**13.** Procédé selon la revendication 11 ou 12, dans lequel la valeur [∅ $CO_2$ mmol/h/$m^2$ de surface corporelle] est de 300 mmol/h/$m^2$ de surface corporelle.

**14.** Procédé selon l'une quelconque des revendications 1 à 7 et 11 à 13, comprenant en outre une ou plusieurs des étapes qui consistent

- à déterminer l'oxydation de protéines chez un individu dans différentes conditions physiques,
- à déterminer la constitution physique générale de l'individu,
- à déterminer l'effort physique moyen ou l'activité physique moyenne de l'individu,
- à déterminer le statut protéique de l'individu sur la base des valeurs d'oxydation de protéines dérivées,
- à déterminer l'oxydation de protéines et le statut protéique des bébés pour déterminer l'état nutritionnel,
- à choisir une nutrition adaptée à l'individu sur la base du statut protéique déterminé en tenant compte de sa constitution physique générale et de son effort physique,
- à donner des conseils nutritionnels pour les diverses catégories, en particulier pour prévenir ou contrer la dénutrition ou à donner un conseil nutritionnel pour bébés pour prévenir ou contrer l'obésité et le développement du cancer à l'âge adulte.

**15.** Procédé selon l'une quelconque des revendications 1 à 7 et 11 à 14, dans lequel la dose initiale totale de $^{13}C$ provient d'un substrat laitier naturellement enrichi en $^{13}C$ isolé selon la revendication 8, 9 ou 10.

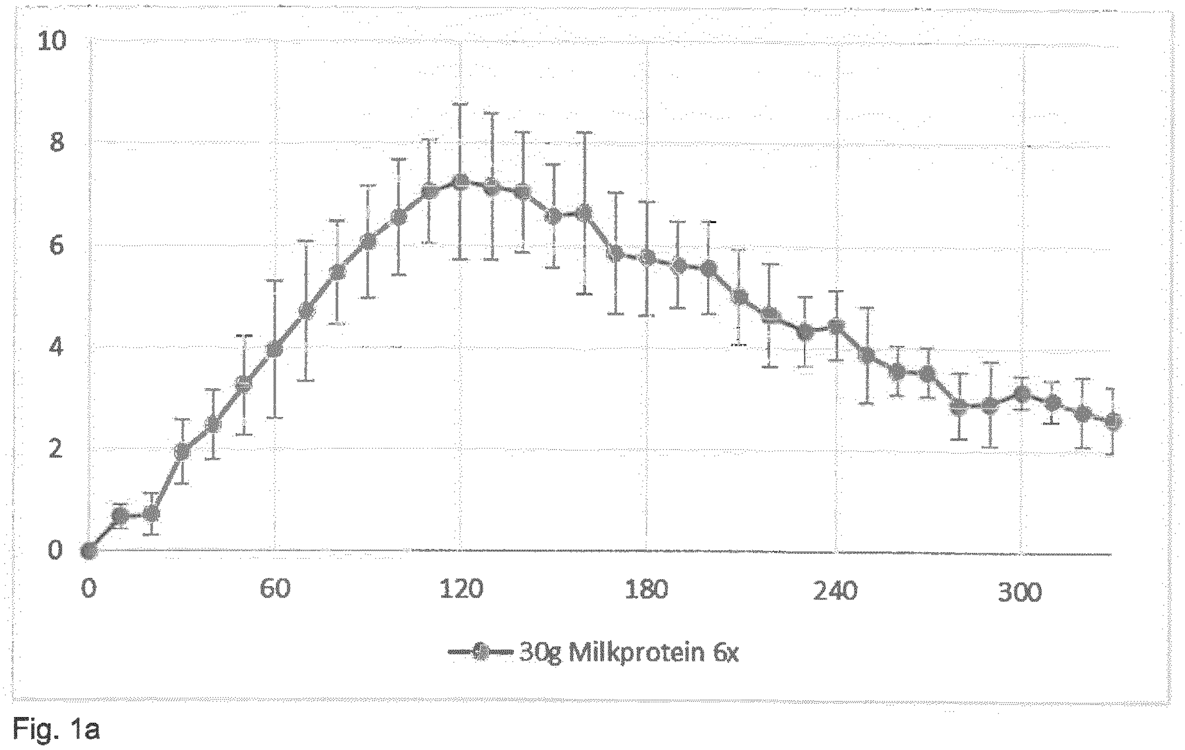
10
8
6
4
2
0
0
60
120
180
240
300
30g Milkprotein 6x

Fig. 1a

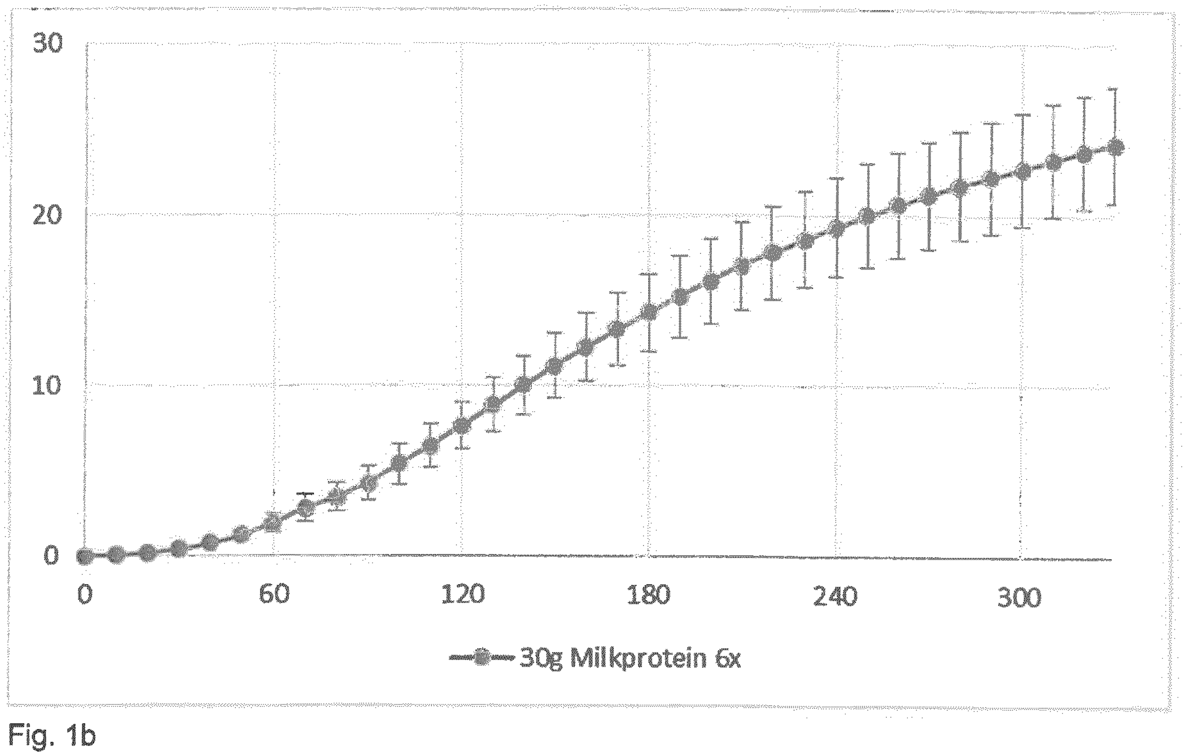
30
20
10
0
0
60
120
180
240
300
30g Milkprotein 6x

Fig. 1b

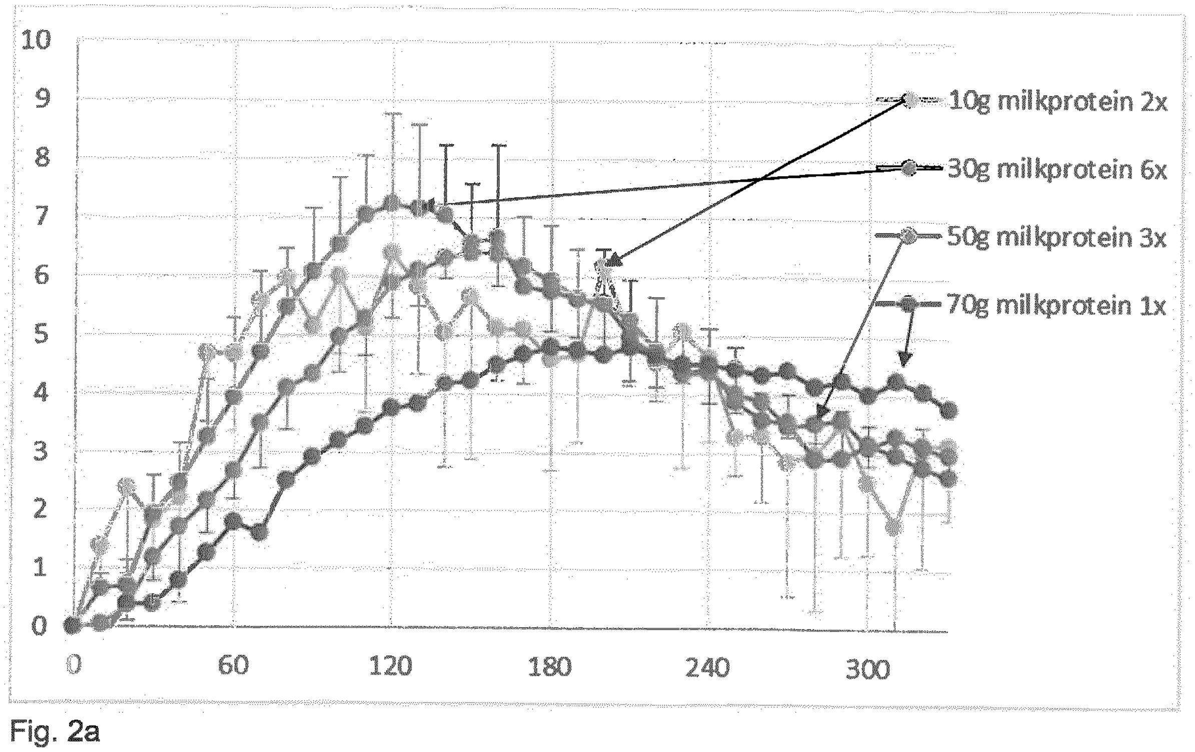
10
9
8
7
6
5
4
3
2
1
0
0
60
120
180
240
300
10g milkprotein 2x
30g milkprotein 6x
50g milkprotein 3x
70g milkprotein 1x

Fig. 2a

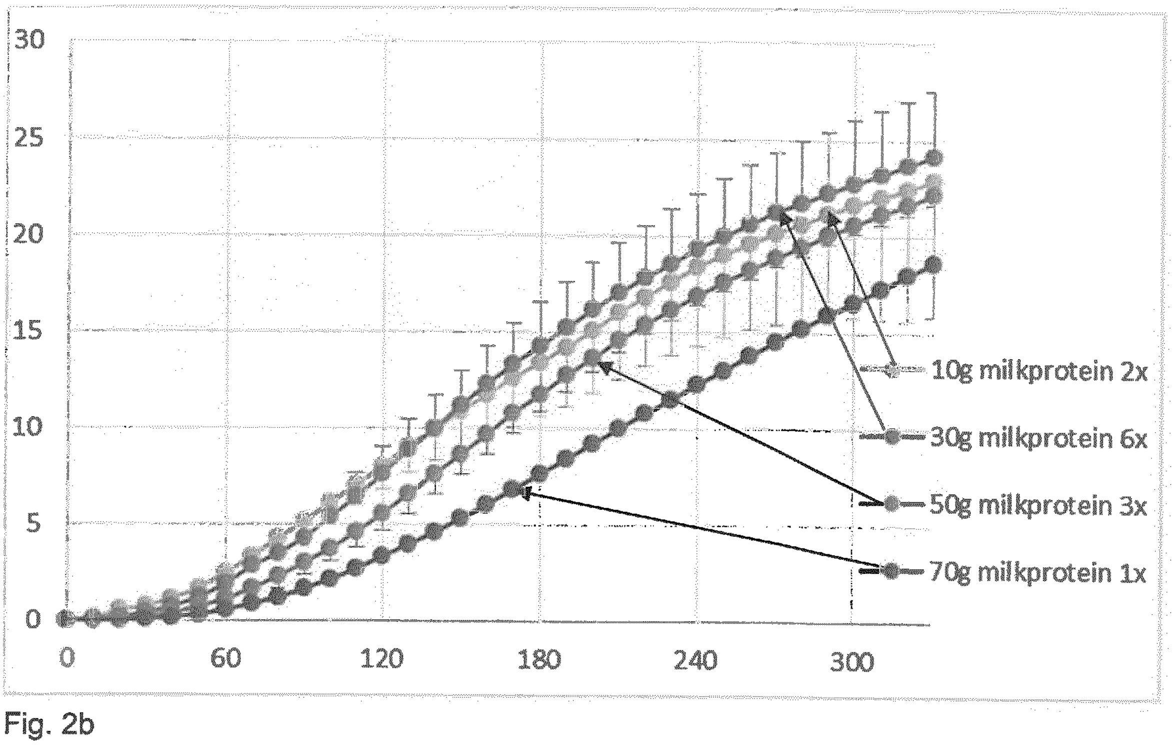
30
25
20
15
10
5
0
0
60
120
180
240
300
10g milkprotein 2x
30g milkprotein 6x
50g milkprotein 3x
70g milkprotein 1x

Fig. 2b

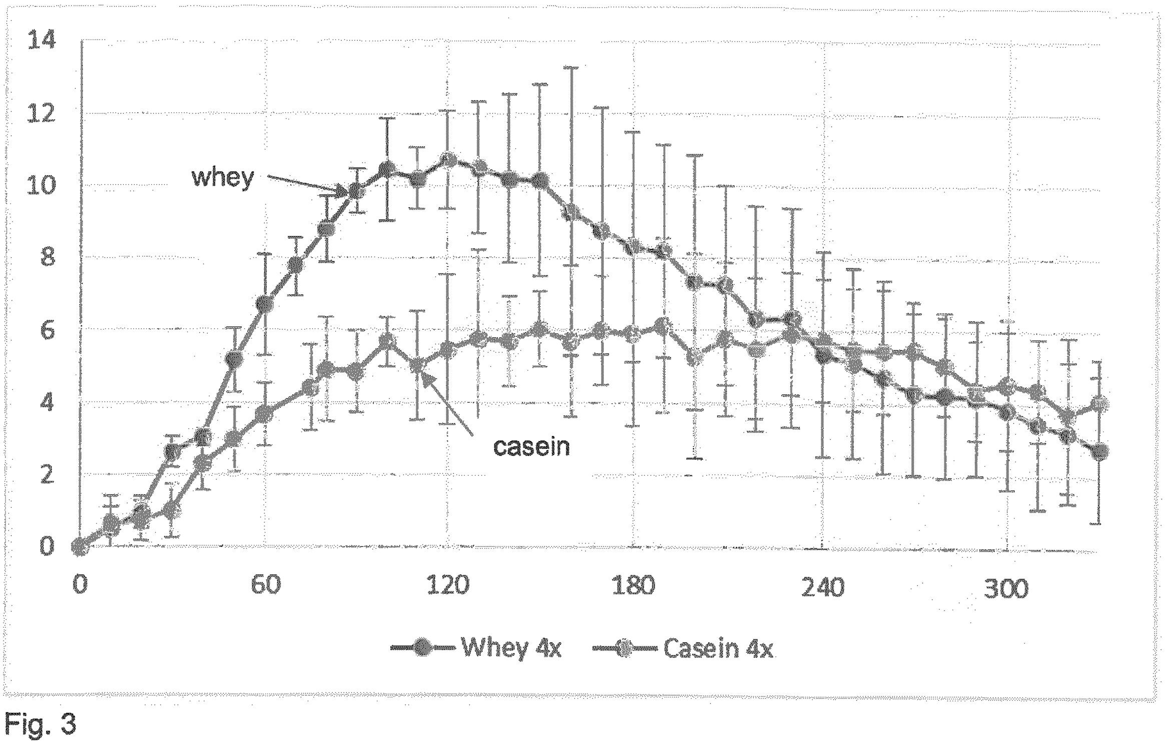
14
12
10
8
6
4
2
0
0
60
120
180
240
300
whey
casein
Whey 4x
Casein 4x

Fig. 3

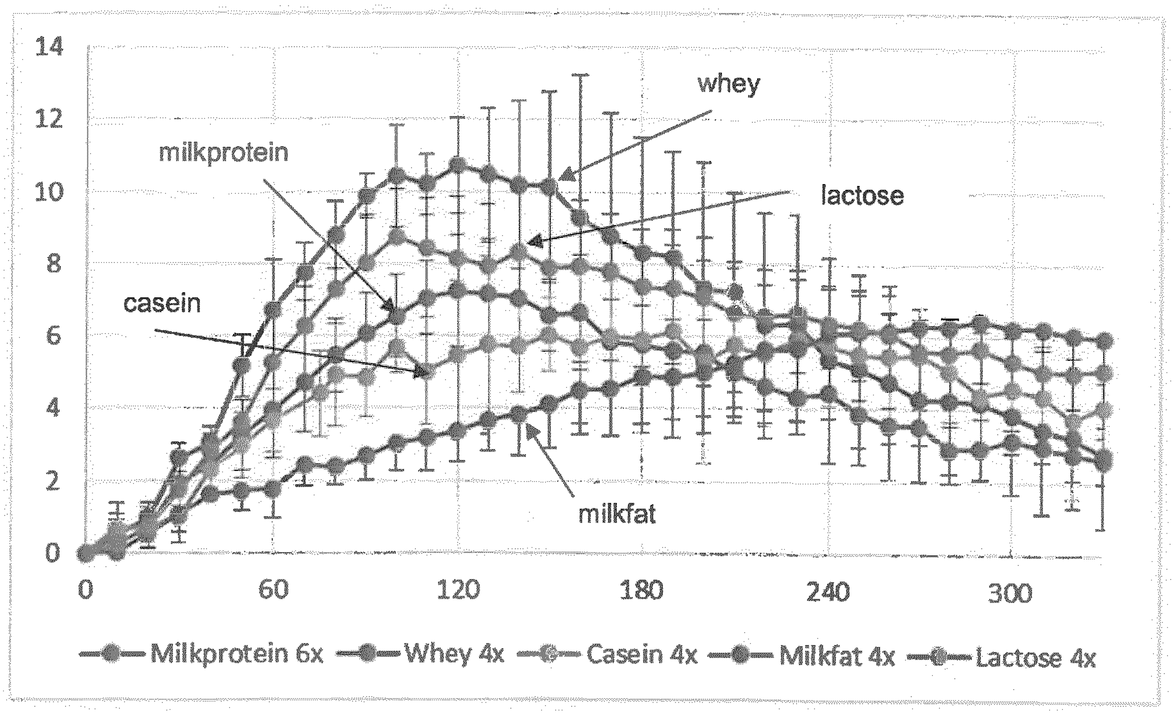
14
12
10
8
6
4
2
0
0
60
120
180
240
300
whey
milkprotein
lactose
casein
milkfat
Milkprotein 6x
Whey 4x
Casein 4x
Milkfat 4x
Lactose 4x

Fig. 4

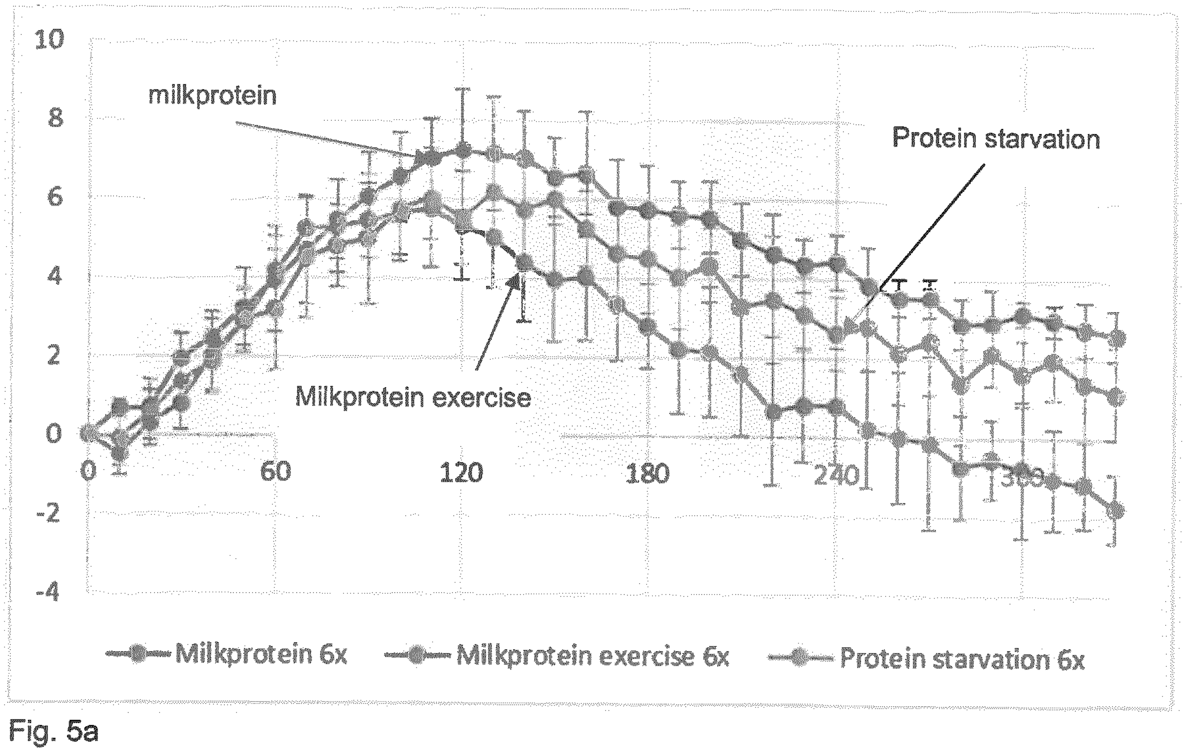
10
8
6
4
2
0
-2
-4
0
60
120
180
240
300
milkprotein
Protein starvation
Milkprotein exercise
Milkprotein 6x
Milkprotein exercise 6x
Protein starvation 6x

Fig. 5a

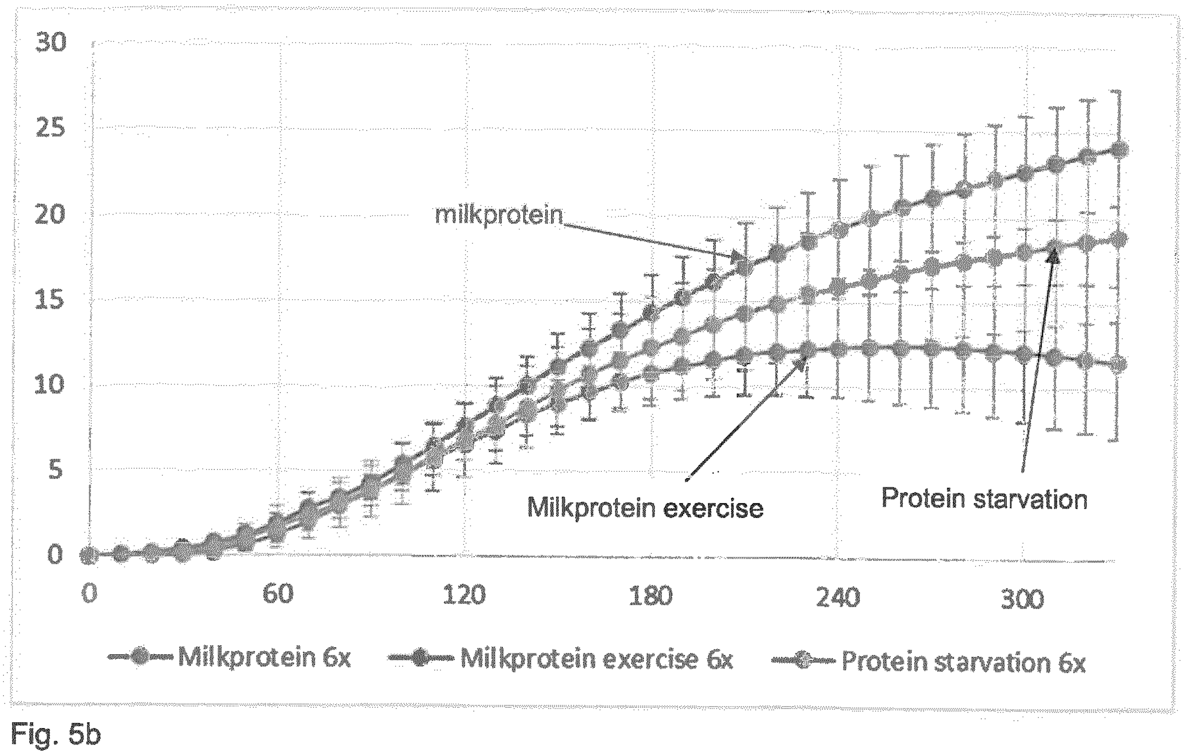
30
25
20
15
10
5
0
0
60
120
180
240
300
milkprotein
Milkprotein exercise
Protein starvation
Milkprotein 6x
Milkprotein exercise 6x
Protein starvation 6x

Fig. 5b

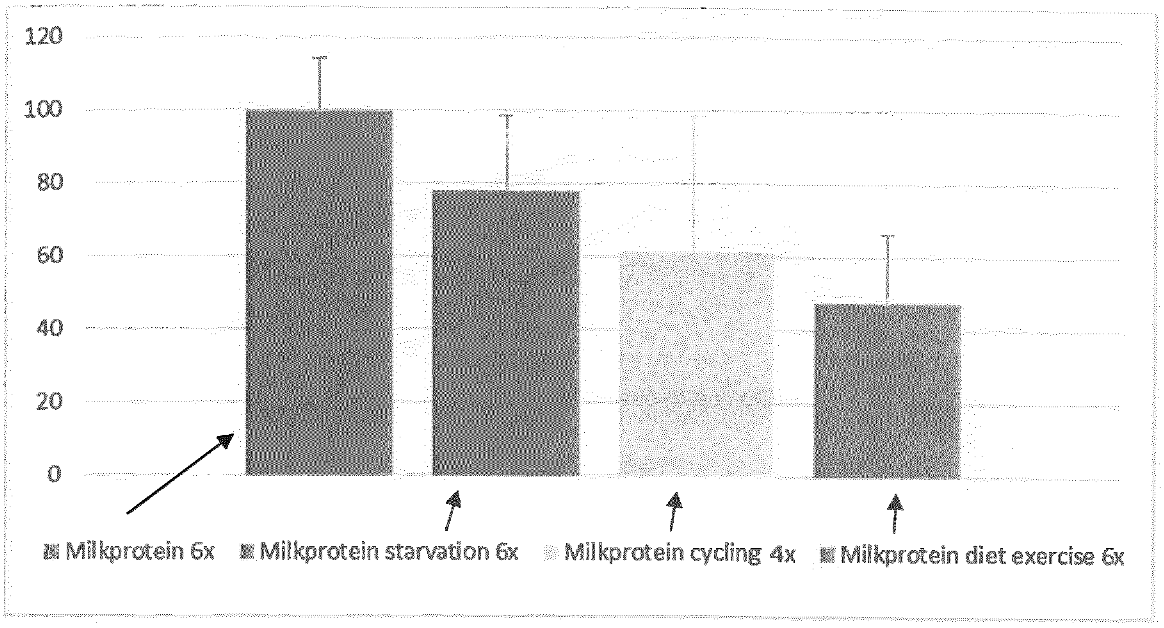
120
100
80
60
40
20
0
Milkprotein 6x
Milkprotein starvation 6x
Milkprotein cycling 4x
Milkprotein diet exercise 6x

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080281194 A


### Non-patent literature cited in the description


- **BRADEN.** *Deutsches Arzteblatt,* 2003, vol. 100, 51-52
- **BOIRIE, Y. et al.** *Physiology,* 1997, vol. 94, 14930-14935
- **EVENEPOEL et al.** *J Nutr,* 1997, vol. 127, 327-331
- **EVENEPOEL et al.** *J Nutr,* 1998, vol. 128, 1716-1722
- **GHOOS Y. et al.** Use of Naturally C-enriched Substrates for the Study of Carbohydrate and Protein Assimilation by Means of CO2 Breath Tests. *Applied Radiation and Isotopes, Int. J. of Radiation Applications and Instrumentation,* 1988, vol. 39 (6), 584
- **Y. GHOOS ; B. BEAUFRERE.** C protein breath tests. *Gut,* 1998, vol. 43 (3), S23-S24
- **METGES et al.** *British Journal of Nutrition,* 1992, vol. 67, 43-55
- **FEDERICA CAMIN et al.** Influence of dietary composition on the carbon, nitrogen, oxygen and hydrogen stable isotope ratios of milk. *Rapid Commun. Mass Spectrom.,* 2008, vol. 22, 1690-1696
- **HAYCOCK GB ; SCHWARTZ GJ ; WISOTSKY DH.** Geometric method for measuring body surface area: A height-weight formula validated in infants, children and adults. *J Pediatr,* 1978, vol. 93, 62-66